# EUROPEAN PATENT APPLICATION

(11) **EP 2 085 082 A1**
(43) Date of publication of application: **05.08.2009**
(21) Application number: 08150755.0
(22) Date of filing: 29.01.2008
(51) Int. Cl.: A61K 31/194, A61P 25/28

(54) **Treatment of cognitive decline**

(71) Applicant: Nutromnia S.R.L., 00135 Rome (IT)
(72) Inventor: Mingrone, Geltrude, 06056, Massa Martana PG (IT)
(74) Representative: Predazzi, Valentina

(57) **Abstract**

The present invention relates to the treatment of decline of cognitive functions, and particularly it relates to the use of medium chain dicarboxylic acids as medicament or a dietary integrator for the treatment of decline of cognitive functions. The dicarboxylic acids according to the present invention have the following formula: HOOC-(CH₂)ₙ-COOH, wherein n is an integer ranging from 6 to 16 or pharmaceutically acceptable salts, derivatives or mixture thereof.

## Description

### Technical field of the invention

The present invention relates to the treatment of decline of cognitive functions, and particularly it relates to the use of medium chain dicarboxylic acids as dietary integrator and as medicament for the treatment of decline of cognitive functions.

### Background of the invention

It is known that cognitive decline exists in different forms. The most severe variety includes dementia, for example Alzheimer disease, frontotemporal dementia and vascular dementia.

An intermediate form consists of "mild cognitive impairment", which represents the cognitive decline preceding diagnosable Alzheimer disease or other forms of dementia and reflects underlying disease-specific pathologic changes.

The third form of mental impairment represents usual cognitive aging. This form normally affects middle-aged and elderly individuals with an assortment of age-associated cognitive problems, not representing early stages of a specific dementing disorder. A primary role in aging and age-related degenerative disorders is played by the progressive increase in endogenously generated reactive oxygen and nitrogen species which cause accumulating damage to cellular lipids, proteins and nucleic acids. Mitochondrial damage may engage an escalating cycle of increasing free radical generation and further damage. This age-associated impairment accounts for a decline in learning and memory, also extending to spatial memory tasks mainly because of the involvement of the hippocampus, which undergoes both functional and morphological changes. Also involved in spatial memory are the neural circuits between the prefrontal cortex and the striatum.

In addition, it is known that diabetes has been associated with encephalopathy and impairments of the cognitive functions and recent studies suggest the existence of a link between diabetes and the probability of developing Alzheimer's disease and other dementias.

Dicarboxylic acids are known as preferential metabolic substrates for the central nervous system. In animals and humans, medium chain dicarboxylic acids, which include mainly decanedioic (sebacic) and dodecanedioic acids, derive from the β-oxidation of longer chain dicarboxylic acids. Long chain dicarboxylic acids, in turn, are formed from the correspondent fatty acids by omega-oxidation in microsomes or are taken in with a diet rich in vegetables.

The publication of Alexander JJ, Snyder A, Tonsgard JH. "Omega-oxidation of monocarboxylic acids in rat brain" Neurochem Res. 1998; 23: 227-33 describes that homogenates of rat brain catalyze the omega oxidation of monocarboxylic acids resulting in dicarboxylic acids with a specific activity remarkably higher than that found in rat liver. Specific activity increases with increasing chain length of the substrate. Cultured rat neurons, astrocytes and oligodendrocytes all contain omega-oxidation activity, and the product of the omega-oxidation in brain almost exclusively consists in dicarboxylic acids.

The publication *"*Medium-chain, even-numbered dicarboxylic acids as novel energy substrates: an update" Nutr Rev. 2006; 64:449-56 reports that oxidation of dicarboxylic acids provide an increase in succinic acid and acetyl-CoA, intermediate substrates of the tricarboxylic acid cycle, which results in an improvement of mitochondrial oxidative efficiency.

It is also generally known that cognitive decline can be primarily prevented by providing dietary supplements like vitamins and/or antioxidants, by replenishing age-depleted hormones such as estrogen, testosterone, or dehydroepiandrosterone, and by promoting healthy lifestyle practices. However, the presently available redressing means do not offer any satisfactory solution of the problem yet.

Therefore, there still exists the need of specific products, compositions and dietary integrators capable of more efficiently preventing and /or treating cognitive decline in mammals and humans. Scope of the present invention is therefore to provide suitable products, compositions and dietary integrators capable of meeting this need.

### Summary of the invention

The present invention is based on the discovery that medium chain dicarboxylic acids (DAs) are provided with pharmaceutical and nutritional activity. When administered orally, they can prevent cognitive decline, specifically age-related and diabetes-related decline.

In fact, the administering of dicarboxylic acids in rats has proven to be useful for age-related and diabetes-related impairment of spatial, reference or working memory.

Moreover, the dicarboxylic acids of the invention can be advantageously used in the treatment of age-related decline of cognitive functions in particular conditions where, for any reasons, it is required to reduce the amount of carbohydrates, fats or proteins, such as in the case of diabetes, chronic renal failure, liver cirrhosis, etc. Under these circumstances the use of dicarboxylic acids is an important source of further energy substrate.

Object of the present invention are therefore dicarboxylic acids of formula HOOC-(CH₂)ₙ-COO wherein n is an integer ranging from 6 to 16, or pharmaceutically acceptable salts, derivatives or mixture thereof for use as a medicament or as dietary integrator, in particular, in the prevention or treatment of decline of cognitive functions.

Another object of the present invention is a composition comprising as active principle a pharmacologically active amount of a substance selected from the dicarboxylic acids of the invention, or pharmaceutically acceptable salts, derivatives or mixture thereof and a pharmaceutically acceptable excipient.

Still another object of the present invention is a dietary integrator comprising a active amount of a substance selected from dicarboxylic acids of the invention or alimentary acceptable salts, derivatives or mixture thereof and a carrier suitable for animal or human alimentation.

Other object will be evident from the present description.

### Detailed description of the invention

The dicarboxylic acids useable according to the present invention are selected from C8 to C18 dicarboxylic acids having formula HOOC-(CH₂)ₙ-COOH, with "n" being an integer ranging from 6 to 16, for instance 1,6-hexanedicarboxylic acid, 1,8-octanedicarboxylic acid, 1,10-decanedicarboxylic acid, 1,12-dodecanedicarboxylic acid, 1,14-tethradecanedicarboxylic acid or 1,16-hexadecanedicarboxylic acid. Preferably decanedioic and dodecanedioic acids are employed for the purpose of the present invention.

The dicarboxylic acids may be used either alone or as a mixture of two or more different acids, for instance C8/C10, C10/C12, C12/C14, C14/C16, C10/C14, C10/C16, C10/C18, C12/C16, C12/C18, C8/C12, C8/C14 or C8/C10/C12, C10/C12/C14. Preferably C10 or C12 will be comprised in the mixture. In binary mixtures, the w/w ratio of the two acids ranges from 10:90 to 90:10, but preferably 50:50. Ternary or higher mixtures will preferably mainly comprise C10 and C12 acids, wherein the sum of C10 and C12 is at least 60% of the mixture, but preferably 70, 80 or 90%.

The dicarboxylic acid of the invention can be used either in the form of free acids or as salts, derivatives or a mixtures thereof. Salts are either inorganic (e.g. Na, K, Ca, Fe, Mg, etc.) or an organic such as ammonium or alkyl ammonium salts or salts with basic amino acids and peptides. Whereas free acids are insoluble in water, salts are soluble in water and aqueous solution. For this reason, salts are sometime the preferred derivative of the dicarboxylic acids.

Derivatives are for example esters, di-esters, amides, di-amides, imides, triglycerides, phospholipids, glycolipids, sfingolipids also resulting by reacting the dicarboxylic acid with one or more amino acids.

The pharmaceutical or nutritional compositions of the invention comprise the dicarboxylic acids with one or more pharmaceutically acceptable excipients. They can be formulated into suitable forms for systemic administration, that is oral, enteral, or parenteral administration.

For oral administration the active agents are formulated preferably into dietary or nutritional supplements or integrators. These compositions may contain the dicarboxylic acids adsorbed on, or admixed with, extracts, oils, such as vegetable (e.g. soy oil) or fish oils or solid carriers such as pellets, beads, granulates, powder suitable for human or animal alimentation. Additionally, they may include vitamins, minerals, stabilizers, flavoring agents and any other known additive. Dietary and nutritional integrators can be packaged into commercial products such as supplemented aliments, snacks, oils, adult and paediatric sip feeds, adult and paediatric tube feeds, nutritional modules. For animal use, poultry and livestock can be fed with dicarboxylic acids in the form of eggs, milk and meat as dicarboxylic acids enriched aliments.

In enteral nutrition, dicarboxylic acids can be administered directly or by tube feeding. Alternatively, soft or hard gelatin capsules, beads, particles, tablets, granulates comprising the dicarboxylic acids and a suitable carrier may be directly used as oral compositions.

Liquid compositions for oral or parenteral use include solutions, suspensions, liposome suspensions, W/O, O/W, W/O/W, O/W/O emulsions. Suitable diluents for this type of administration are: water, saline solution, sugar solutions, hydro-alcohol solutions, oily diluents such as vegetable oils, mineral oils, polyoils, such as glycerol, ethylene or polypropylene glycol, or any other diluents compatible with the administration method in terms of sterility, pH, ionic strength and viscosity.

In the case of emulsions or suspensions the composition may contain suitable surfactant agents commonly used in the formulation of pharmaceutical or alimentary compositions.

Compositions with delayed release may also be used, which either include polymers such as polylactate, poly(meth)acrylate, polyvinyl-pyrrolidone, methylcellulose, carboxymethylcellulose or consist of subcutaneous implants based, for example, on polylactate or other biodegradable polymers.

In the compositions or dietary integrator according to the present invention the pharmacologically active amount of the dicarboxylic acids, salts or derivative thereof ranges from 1% to 20% of the composition calculated on the free acid weight basis. For instance amounts of 2%, 5%, 7%, 10% or 15% can advantageously be used.

The activity of the claimed compositions or alimentary integrators may easily be assessed by way of well known tests.

Since it is known that in humans as in rats, senescence impairs spatial memory, a number of tests and animal models have been developed in the past for the assessment of spatial memory ability and for checking the presence and the progression of cognitive decline. Results reported on animal models proved predictable for human behaviour.

Suitable known animal tests of spatial learning and memory are the platform, the radial arm maze and the Morris water maze, which is a particularly useful tool. Furthermore, the water maze is well suited for testing older rats because the motivation stimulus, i.e. escape from water, does not require the food or water deprivation that is used in appetitive tasks like the radial arm maze or T maze. Reference memory and working memory are two types of memory that have been largely studied in young rats. While reference memory is trial independent and is required to learn the general rules of any task, working memory is, on the other hand, trial dependent and has a major temporal component since it is necessary to learn both the type of stimulus presented and the time of stimulus presentation; working memory is distinguished from reference memory by the transient nature of the stimulus-response relationship (see Neurobiology of Aging, vol.16, no.2, pp 149-160,1995: Frick K.M., Baxter M.G., Markowska A.L., Olton D.S. and Prince D. "Age-related spatial reference and working memory deficits assessed in the water maze"; Behav Brain Res. 2007;180:28-41: Moreira T, Malec E, Ostenson CG, Efendic S, Liljequist S. "Diabetic type II Goto-Kakizaki rats show progressively decreasing exploratory activity and learning impairments in fixed and progressive ratios of a lever-press task".

As shown in the following experimental section, the dicarboxilic acids of the invention, with shorter or longer chain length, are useful in preventing and treating cognitive decline in a series of pathological conditions as well as in physiological age-related form.

The invention is described hereafter in all its details by means of examples which serve purely illustrative purposes and in no way limit the invention.

### Experimental work

### Age-related cognitive decline experiments

In order to evaluate the impact of a diet enriched with dicarboxylic acids on age-related cognitive decline, 20 Wistar rats of both sexes (half male and half female), 4 months old and 23 months old were used.

Rats of the same sex were housed (2-3 per cage) in a colony room with a 12:12 light- dark cycle and ad libitum access to food and water. All behavioural testing occurred during the light phase of the light- dark cycle. Body weight was recorded twice a week.

The rats were divided in two groups of 10. The rats of a group (treated group) were fed with pellets enriched with 5% dodecanedioic acid, the rats of the other group being the control group. The dietary supplementation was carried out for 3 months and in the last week of treatment both groups underwent some tasks in the Morris water maze in order to test their cognitive abilities.

The choice accuracy of treated rats, i.e. the time to find the platform or, in other terms, the time for correct choice swim, was significantly lower than in controls (19.6 ± 1.6 sec vs. 23.7 ± 4.2 sec ; P = 0.01) and it did not differ significantly with respect to young rats (18.2±1.7 sec). Individual data are reported in Table 1.

**TABLE 1: Swimming time (sec.)**

| CONTROLS | C12 | YOUNG RATS |
|---|---|---|
| 31 | 18 | 16 |
| 22 | 22 | 19 |
| 27 | 19 | 19 |
| 25 | 18 | 15 |
| 23 | 21 | 19 |
| 20 | 20 | 20 |
| 29 | 18 | 18 |
| 18 | 18,5 | 17 |
| 22 | 19,6 | 20 |
| 20 | 21,8 | 19 |

| **Average values** | | |
|---|---|---|
| **23,70** | **19,59** | **18,20** |
| **± 4,22** | **± 1,56** | **± 1,69** |

The treatment with dodecanoic acid significantly improved the travelled distance of old rats (409.5± 98.6 cm vs. 520.5± 74.6 cm; P =0.011), up to becoming comparable with the values found in 4-months old rats (338.0± 82.3 cm, P = NS). Individual data are reported in Table 2.

**TABLE 2 Travelled distance (cm)**

| CONTROLS | C12 | YOUNG RATS |
|---|---|---|
| 600 | 400 | 350 |
| 500 | 300 | 280 |
| 400 | 580 | 450 |
| 450 | 450 | 350 |
| 550 | 300 | 250 |
| 580 | 520 | 500 |
| 600 | 350 | 350 |
| 420 | 325 | 300 |
| 575 | 500 | 300 |
| 530 | 370 | 250 |

| **Average values** | | |
|---|---|---|
| **520,50** | **409,50** | **338,00** |
| **± 74,63** | **± 98,56** | **± 82,30** |

The repeated acquisition task proved a net improvement of the travelled distance in all the old rats, although treated rats performance remained better than in non treated mates (327.0±80.4 vs. 394.0±55.2 cm, P = 0.04) (see Table 3).

**TABLE 3:**

| Travelled distance (cm) in repeated trials | |
|---|---|
| CONTROLS | C12 |
| 400 | 300 |
| 350 | 200 |
| 320 | 350 |
| 400 | 410 |
| 490 | 260 |
| 450 | 470 |
| 430 | 300 |
| 320 | 280 |
| 410 | 400 |
| 370 | 300 |

| **Avarage values** | |
|---|---|
| **394,00** | **327,00** |
| **± 55,22** | **± 80,42** |

The time of permanence in the correct quadrant was 41.7±5.2% sec in C12 treated rats with respect to 36.9±4.8% sec. in controls (P = 0.04), and it was not significantly different from the value found in young rats (46.0±5.2% sec) (Table 4).

**TABLE 4: time in the correct quadrant %**

| CONTROLS | C12 | YOUNG RATS |
|---|---|---|
| 38 | 50 | 55 |
| 30 | 45 | 45 |
| 35 | 40 | 42 |
| 42 | 42 | 50 |
| 30 | 35 | 51 |
| 37 | 44 | 40 |
| 40 | 48 | 45 |
| 45 | 37 | 42 |
| 37 | 41 | 50 |
| 35 | 35 | 40 |

| **Average values** | | |
|---|---|---|
| **36,90** | **41,70** | **46,00** |
| **± 4,77** | **± 5,17** | **± 5,21** |

### Diabetes-related cognitive decline experiments

In order to evaluate the impact of a diet enriched with dicarboxylic acids on diabetes-related cognitive decline, 10 Wistar rats and 20 Goto-Kakizaki rats of both sexes (half male and half female), aged 4 months were used.

Rats of the same sex were housed (2-3 per cage) in a colony room with a 12:12 light- dark cycle and ad libitum access to food and water. All behavioral testing occurred during the light phase of the light- dark cycle. Body weight was recorded twice a week.

Ten Goto-Kakizaki rats (treated group) were selected at random from the initial group of 20 animals and were fed with pellets enriched with 5% dodecanedioic acid. The remaining ten Goto-Kakizaki rats formed the control group and received standard Purina chow. A group of ten Wistar rats was also fed with standard Purina chow, as second control group.

The dietary supplementation was carried out for 1 month and in the last week of treatment the different groups underwent some tasks in the Morris water maze in order to test their cognitive abilities.

The choice accuracy, i.e. the time to find the platform of treated Goto Kakizaki rats was significantly lower than that of control diabetic rats (23.24±2.81 sec vs. 27.84±2.77 sec; P = 0.0017) and it did not differ significantly from that of Wistar rats matched by age (21.21±2.01 sec) (Table 5) .

**TABLE 5:CHOICE ACCURACY (sec)**

| Goto | Goto C12 | Wistar |
|---|---|---|
| 24,3 | 22,6 | 20 |
| 31,5 | 23,4 | 19,4 |
| 28 | 25,9 | 24,2 |
| 24,2 | 27 | 21,4 |
| 29 | 24 | 19,8 |
| 30,7 | 22,1 | 22,1 |
| 27,4 | 23,5 | 23,7 |
| 26,5 | 26,2 | 21,5 |
| 25,4 | 19,3 | 22,3 |
| 31,4 | 18,4 | 17,7 |

| **Average values** | | |
|---|---|---|
| **27,84** | **23,24** | **21,21** |
| **± 2,78** | **± 2,813** | **± 2,01** |

The travelled distance of diabetic rats was significantly improved from 548.9±91.34 to 423.6±83.82 cm (P = 0.005) in the group receiving dodecanoic acid and it was not statistically different from that of the control group (369.3±59.48 cm; P = n.s.) (Table 6).

**TABLE 6: Travelled distance (cm)**

| Goto | Goto C12 | Wistar |
|---|---|---|
| 600 | 418 | 350 |
| 630 | 511 | 315 |
| 477 | 368 | 369 |
| 524 | 354 | 421 |
| 431 | 477 | 478 |
| 578 | 541 | 311 |
| 629 | 326 | 326 |
| 394 | 511 | 448 |
| 671 | 424 | 321 |
| 555 | 306 | 354 |

| **Average values** | | |
|---|---|---|
| **548,90** | **423,60** | **369,30** |
| **± 91,34** | **± 83,82** | **± 59,48** |

A net improvement of the travelled distance in the repeated acquisition task was observed in all diabetic rats, although dodecanoic acid treated rats performance remained better than that of non treated mates (498.9±93.06 vs. 374.1±78.23 cm, P = 0.004) (Table 7).

**TABLE 7:**

| REPEATED ACQUISITION TASK TRAVELLED DISTANCE (cm) | |
|---|---|
| Goto | GotoC12 |
| 400 | 370 |
| 515 | 359 |
| 462 | 300 |
| 500 | 291 |
| 402 | 444 |
| 555 | 498 |
| 598 | 300 |
| 370 | 476 |
| 663 | 403 |
| 524 | 300 |

| **Average values** | |
|---|---|
| **498,90** | **374,10** |
| **± 93,06** | **± 78,23** |

The time of permanence in the correct quadrant was 44.0 ± 4,1 sec in dodecanoic acid treated rats with respect to 33.2 +2.7 sec in controls (p<0.0001), and it was not significantly different from the value found in Wistar rats (46.6 ±4.3 sec) (Table 8).

**TABLE 8:**

| time of permanence in the correct quadrant (sec) | | |
|---|---|---|
| Goto | Goto C12 | Wistar |
| 34 | 50 | 52 |
| 32 | 46 | 51 |
| 36 | 41 | 44 |
| 33 | 39 | 49 |
| 38 | 47 | 43 |
| 29 | 45 | 48 |
| 31 | 44 | 39 |
| 31 | 38 | 42 |
| 35 | 41 | 48 |
| 33 | 49 | 50 |

| **Average values** | | |
|---|---|---|
| **33,20** | **44,00** | **46,60** |
| **± 2,66%** | **± 4,14%** | **± 4,33%** |

### Water maze apparatus

A large metal pool (180 cm in diameter) filled with opaque water and kept at a constant temperature 24 ± 1°C and a 10 cm platform were used in place discrimination and visual discrimination tasks. A straight plastic alley (10 × 70 cm) was placed in the large pool and used during the straight swim test to estimate swimming ability. A video-camera was used to record the different phases of the experiments.

### Place discrimination task

Each rat received 1 day of training with three sessions of five platform trials and one probe trial each and a random order of start positions. During the platform trials, the platform was submerged but accessible to the rat, whereas during the probe trials, the platform was collapsed at the bottom of the tank for variable intervals (from 10 to 40 sec) to test the subject's preference for the platform location. The collapsed platform was then returned to its raised position at the end of the probe trial to maintain the same response-reinforcement contingency as in the platform trials. The percentage of time spent in the correct quadrant, the choice accuracy [i.e. the time in seconds to find the platform], and the travelled distance or distance swum to find and climb on the platform [in centimeters] were measured. A a shorter time to find the platform indicate better performance. Higher scores for a percentage of time spent in the correct quadrant reflected better performance. Location of the platform and a set of spatial cues around the tank were changed between the testing periods.

### Repeated acquisition task

The training started on the day after the place discrimination task and consisted of 1 day of training with two sessions of 9 trials each. During the first session, the set of extra maze cues, as well as the location of platform, was different from that on the previous place discrimination task. During the second session, the platform was moved to the new position, with the set of external cues remaining the same. Each session consisted of eight platform trials and one probe trial. The measures of platform and probe trials were the same as in the place discrimination task.

### Statistical Analysis

Data are reported as mean ± SD. Comparison between groups was made by repeated-measures ANOVA. Body weight was used as a covariance to adjust the significance of between-group differences in speed of swimming. P<0.05 was considered as significant.

### Conclusions

The results of the present investigation show that dodecanoic acid, administered orally, ameliorates both age-related and diabetes-related decline in various cognitive functions in rats. This improvement reached a level of choice accuracy equivalent to that observed in the young rats or in non diabetic animals, in age-related and diabetes-related cognitive decline studies respectively.

Although a protective effect of training against aging-related decline in the repeated acquisition task was found, yet the treated rats had a still better performance than the control mates. Similar results were also obtained in diabetic rats fed with dodecanoic acid-supplemented food.

## Claims

1. Dicarboxylic acids having formula HOOC-(CH₂)ₙ-COOH, wherein n is an integer ranging from 6 to 16 or pharmaceutically acceptable salts, derivatives or mixture thereof for use as a medicament.

2. Dicarboxylic acids according to claim 1 wherein n is 8 or 10 or pharmaceutically acceptable salts, derivatives or mixture thereof.

3. Dicarboxylic acids according to claims 1 or 2 or pharmaceutically acceptable salts or derivatives or mixture thereof for use in the prevention or treatment of cognitive decline.

4. Dicarboxylic acids according to claims 1 or 3 or pharmaceutically acceptable salts or derivatives or mixture thereof, wherein the cognitive decline is age-related cognitive decline or diabetes-related cognitive decline.

5. Dicarboxylic acids according to any one of claims 1 to 4, wherein the age-related cognitive decline is age related impairment of spatial, reference or working memory.

6. Dicarboxylic acids according to any one of claims 1 to 5, wherein derivatives are selected from esters, di-esters, amides, di-amides, imides, mono-, di- or triglycerides, phospholipids, glycolipids, sfingolipids or derivatives resulting by reacting the dicarboxylic acid with one or more amino acids or peptides.

7. Dicarboxylic acids according to any one of claims 1 to 6, wherein the salt is an inorganic or organic salt.

8. A pharmaceutical composition comprising, as active principle, a pharmacologically active amount of one or more dicarboxylic acids according to any one of claims 1 to 7, or pharmaceutically acceptable salts, derivatives or mixture thereof and a pharmaceutically acceptable excipient.

9. The composition according to claim 8, wherein the active principle is selected from decanedioic or dodecanedioic acids or salts, derivatives or mixture thereof.

10. The composition of claims 8 or 9 comprising an amount of one or more dicarboxylic acids, salts, derivatives or mixtures thereof ranging from 1% to 20%, calculated on the free acid weight basis.

11. The composition according any one of claims 8 to 10, formulated for oral or enteral, or parenteral administration.

12. Dietary integrator comprising, as active principle, an active amount of one or more dicarboxylic acids according to any one of claims 1 to 7, or a salts, derivatives or mixture thereof and a carrier suitable for animal or human alimentation.

13. Dietary integrator according to claim 12, wherein the active principle is selected from decanedioic or dodecanedioic acids or salts, derivatives or mixture thereof.

14. Dietary integrator according to claims 12 or 13 comprising an amount of one or more dicarboxylic acids, salts, derivatives or mixtures thereof ranging from 1% to 20%, calculated on the free acid weight basis.

15. Dietary integrator according to claims 12 or 14 formulated in the form of a supplemented aliment, a snack, drinks or adult and paediatric sip feeds, adult and paediatric tube feeds, nutritional modules.

16. Method of preparation of the compositions or the integrator according to any one of claims 8 to 15 comprising admixing a pharmacological active amount of the one or more dicarboxylic acids with a suitable excipient or carrier.

17. Use of the dicarboxilic acids according to any one of claims 1 to 6 for the preparation of a medicament or a dietary integrator for preventing or treating cognitive decline.
